# EUROPEAN PATENT APPLICATION

(11) **EP 4 062 897 A1**
(43) Date of publication of application: **28.09.2022**
(21) Application number: 20890137.1
(22) Date of filing: 23.11.2020
(51) Int. Cl.: A61K 8/60, A61Q 7/00, A61Q 5/00, A61Q 19/08

(54) **HAIR CARE COMPOSITION AND PREPARATION METHOD THEREFOR**

(30) Priority: 21.11.2019 CN 201911146375
(71) Applicant: BioRight Worldwide Company Limited, Road Town, Tortola (VG)
(72) Inventor: LO, Kam Chun, Hong Kong Science Park, Shatin, New Territories, Hong Kong (CN); WANG, Jun, Hong Kong Science Park, Shatin, New Territories, Hong Kong (CN); POON, Wing Keung, Hong Kong Science Park, Shatin, New Territories Hong Kong (CN)
(74) Representative: Cohausz & Florack
(86) International application number: PCT/CN2020/130814
(87) International publication number: WO 2021/098874

(57) **Abstract**

Disclosed are a hair care composition and a preparation method thereof. The hair care composition comprises nicotinamide mononucleotide or a salt thereof or a hydrate thereof, and a base ingredient for stabilizing the nicotinamide mononucleotide or the salt thereof or the hydrate thereof. The hair care composition can improve the stability of nicotinamide mononucleotide in liquid state, and bring out a brand-new solution to the problems concerning aging and growth of hair and the stability of nicotinamide mononucleotide in liquid state, and bring out a breakthrough in the diversification of the application manners of nicotinamide mononucleotide.

## Description

### TECHNICAL FIELD

The present invention belongs to the field of personal care and specifically relates to a hair care composition containing nicotinamide mononucleotide or a salt thereof or a hydrate thereof.

### BACKGROUND

Recent biochemical studies have found that the level of coenzyme I in the human body decreases gradually with age. Relevant studies also suggest that the decrease of coenzyme I level is closely related to slow down the body metabolism, decline of cellular functions to cause the related diseases such as diabetes, or the development of cancer due to a loss of cellular auto-repair function. In view of the above, replenishing the level of coenzyme I in the body is a direct and effective solution. Clinical studies on the mechanism, animal and pathology of supplementation of coenzyme I have been conducted in China and abroad, and they all confirm that supplementation of coenzyme I is effective in improving the above health status, and it has been confirmed in animal experiments that this method can improve a locomotor ability of aged mice; on the contrary, the locomotor ability of control mice is weaker in old age.

Nicotinamide mononucleotide (NMN) is the most direct precursor for converting to coenzyme I (Nicotinamide adenine dinucleotide, NAD+) in human body. Studies have demonstrated that nicotinamide mononucleotide is the most direct way to rapidly increase the level of coenzyme I in human organs and cells. The coenzyme I is mainly responsible for the transport of hydrogen ions (H-) in biological reactions of cells in the body, and is essential for regulating biological reactions such as metabolism, gene regulation and even regulating a physiological clock. Thus, the level of coenzyme I in cells has an upclose relationship with health.

Hair growth is also closely related to coenzyme I. Recent studies in hair growth projects have found that a synthesis of lactic acid and levels thereof in hair follicle cells are critical for initiating the hair growth cycle, while researchers have found that hair follicle cells also require high levels of ATP to maintain their normal function. Coenzyme I and its reduced coenzyme I are important raw materials for the synthesis of lactic acid in hair follicle cells: when the level of coenzyme I in cells decreases, lactic acid cannot be synthesized and the level thereof decreases, resulting in the suspension of hair growth.

Coenzyme I is also an essential substrate for ATP synthesis in the cellular mitochondria: a decrease in coenzyme I also causes the glands in the hair follicle cells to be unable to synthesize enough ATP such that it is difficult to support daily functioning of the cells. In the absence of both lactic acid and ATP in the hair follicle cells, hair growth inevitably tends to slow down or even falls out, and with the fading follicles, the number of pigment cells decreases accordingly, leading to graying of the hair color. All of these phenomena and the decline of body functions are the consequences of aging, which are caused by the decrease of coenzyme I in the body. Restoration of the level of coenzyme I in the body can be done through exercise and control of caloric intake, but the effects vary from person to person and are not sustainable. Oral administration of nicotinamide mononucleotide supplements provides an easy and direct way to do so and is effective in boosting the overall level of coenzyme I in the body. Although oral administration of nicotinamide mononucleotide supplements can significantly improve and enhance all health conditions in the body, it cannot target hair or hair problems to provide a cosmetic improvement to the hair on one's head or other parts of the body in the shortest possible time. In order to solve these problems, the best solution is to develop a hair care composition with nicotinamide mononucleotide as the main active functional ingredient.

The hair care composition must be effective to be evenly applied over the skin such that the main active ingredient of the care composition, nicotinamide mononucleotide, can contact the epidermis and hair follicles and directly reach the cells. To achieve this, the hair care composition must be mainly in liquid form with different viscosities. However, the stability of nicotinamide mononucleotide in liquid form is poor, with a typical half-life of 14 days in aqueous solution. Due to the instability of nicotinamide mononucleotide in liquid state, there are technical difficulties for nicotinamide mononucleotide to be used in related applications. This is also the main reason for the failure to produce and launch related products in the market yet.

### SUMMARY

In order to solve at least one of the above problems, the present invention provides a hair care composition and a method of preparing the same. The hair care composition of the present invention provides a solution to enhance the stability of nicotinamide mononucleotide in the liquid state, sets a novel direction to solve the problems concerning aging and growth of hairs and the stability of nicotinamide mononucleotide in the liquid state, and brings out a breakthrough in the diversification of the application manners of nicotinamide mononucleotide.

Specifically, the present invention provides:
A hair care composition comprising nicotinamide mononucleotide or a salt thereof or a hydrate thereof, and a base ingredient for stabilizing the nicotinamide mononucleotide or the salt thereof or the hydrate thereof.

The nicotinamide mononucleotide or the salt thereof or the hydrate thereof is in solid, liquid or atomized form.

The base ingredient for stabilizing the nicotinamide mononucleotide or the salt thereof or the hydrate thereof comprises one or more of an anionic surfactant, an amphoteric surfactant, a cationic surfactant, an antioxidant, a pH buffer and an acidity regulator.

The hair care composition is in the form of any one of a shampoo, conditioner, serum, soap, spray, cream, hair wax, gel and paste.

The hair care composition is any one of an eyebrow care product, an eyelash care product and a beard care product.

The base ingredient comprises an ingredient for preventing degradation of nicotinamide mononucleotide in liquid or atomized form, such as one or more selected from sialic acid, theanine, beta-carotene and sodium phosphate.

The nicotinamide mononucleotide or the salt thereof or the hydrate thereof represents from 0.01 wt% to 25 wt%, preferably from 0.1 wt% to 20 wt%, and more preferably from 0.1 wt% to 15 wt% of a total weight of the hair care composition, on a basis of nicotinamide mononucleotide.

The hair care composition further comprises water.

Optionally, the water is present in an amount of 50-95 wt%, preferably 60-92 wt%, and more preferably 65-90 wt%, based on a total weight of the hair care composition.

Relative to the total weight of the hair care composition,
the anionic surfactant is present in an amount of 15-65 wt%, preferably 25-45 wt%, and more preferably 30-40 wt%;
optionally, the amphoteric surfactant is present in an amount of 1-15 wt%, preferably 1.5-8 wt%, and more preferably 2-7 wt%;
optionally, the cationic surfactant is present in an amount of 0.01-0.09 wt%, preferably 0.02-0.08 wt%, and more preferably 0.03-0.08 wt%;
optionally, the antioxidant is present in an amount of 0.5-3 wt%, preferably 0.9-2.5 wt%, and more preferably 1.5-2 wt%;
optionally, the acidity regulator is present in an amount of 0.05-1 wt%, preferably 0.2-0.85 wt%, and more preferably 0.3-0.7 wt%; and
optionally, the pH buffer is present in an amount of 1-3 wt%, preferably 1.5-2.5 wt%, and more preferably 2-2.5 wt%.

The anionic surfactant is selected from at least one of sodium laureth sulfate, sodium lauryl sulfate, ammonium lauryl sulfate, sodium methyl cocoyl taurate, lauryl polyether-5 carboxylic acid, sodium lauryl glucose carboxylate and mixtures thereof.

The amphoteric surfactant is at least one of an alkyl amphoteric carboxylate, an alkyl amino acid salt, cocamidopropyl betaine, cocamide monoethanolamine, lauryl glucoside, coco methyl dextran or mixtures thereof.

The cationic surfactant is at least one selected from polyquaternium-10, guar hydroxypropyltrimethylammonium chloride or mixtures thereof.

The antioxidant is at least one selected from L-cysteine, DL-methionine, vitamin A, vitamin C, vitamin E, sodium phytate or mixtures thereof.

The acidity regulator is at least one selected from citric acid, oxalic acid, lactic acid, hydrochloric acid or mixtures thereof.

The eyebrow care product is an eyebrow care liquid, an eyebrow care spray and an eyebrow care cream; the eyelash care product is an eyelash care liquid assembly; and the beard care product is a beard care liquid, a beard care spray and a beard care cream.

The hair care composition further comprises a functional additive or additives for enriching an appearance, texture, odor and user experience of the hair care composition.

The functional additive or additives is/are selected from sensitizers, foam enhancers, fragrance agents, functional excipients or any combinations thereof.

The present invention also provides a method for preparing a hair care composition, comprising the steps of
providing nicotinamide mononucleotide or a salt thereof or a hydrate thereof,
providing an aqueous solution of a base ingredient for stabilizing the nicotinamide mononucleotide or the salt thereof or the hydrate thereof in a liquid or gaseous state; and
mixing the nicotinamide mononucleotide or the salt thereof or the hydrate thereof with the aqueous solution and adjusting the pH to obtain the hair care composition.

The base ingredient for stabilizing the nicotinamide mononucleotide or the salt thereof or the hydrate thereof comprises one or more of an anionic surfactant, an amphoteric surfactant, a cationic surfactant, a nonionic surfactant, an antioxidant, a pH buffer and an acidity regulator.

The method comprises:
(1) mixing one or more anionic surfactants to obtain a first type of surfactant,
(2) mixing one or more amphoteric surfactants to obtain a second type of surfactant,
(3) optionally, mixing one or more cationic surfactants to obtain a third type of surfactant,
(4) mixing the first type of surfactant, the second type of surfactant and optionally the third type of surfactant to obtain a complex surfactant,
(5) adding nicotinamide mononucleotide to the complex surfactant to obtain a mixture,
(6) adding a pH buffer to the mixture and adjusting the mixture to a pH of 4.85-7.0 using an acidity regulator so as to obtain a hair care composition; and
(7) adding an antioxidant to the mixture, thereby obtaining the hair care composition.

The present invention has at least one of the following advantages and positive effects.
1. The present invention provides a hair care composition comprising nicotinamide mononucleotide as the main active ingredient and capable of maintaining the activity of nicotinamide mononucleotide or a salt thereof or a hydrate thereof stably over a long period of time for supplying nicotinamide mononucleotide to the mitochondria in the hair follicle stem cells and epidermal cells, thereby elevating the level of coenzyme I in hair follicle stem cells and epidermal cells, inducing normal energy production of the cells and activating the vitality of the cells.
2. Compared with nicotinic acid, nicotinamide and other derivatives or the like, nicotinamide mononucleotide used in the present invention is the immediate precursor of coenzyme I. It is directly converted into coenzyme I after cellular uptake and is not limited by the multiple enzymatic reactions in vivo. Meanwhile, nicotinamide mononucleotide is a small molecule, which can be rapidly absorbed by human body and reach the epidermal cells.
3. The hair care composition of the present invention can directly raise the level of coenzyme I for hair follicle stem cells and epidermal cells. Compared with oral administration of nicotinamide mononucleotide supplements only, the practice of the present invention is more significant in addressing the hair problems and can show more obvious effects in a shorter time. Therefore, the present invention is suitable for middle-aged and elderly people who are concerned about hair loss and gradual graying of hair color, or the people with slow and thin hair growth, as well as for the people who are concerned about hair health in general.

### BRIEF DESCRIPTION OF DRAWINGS

FIG. 1 illustrates the comparative results of the stability of Example 1, Example 15, Example 16 and 0.5% aqueous nicotinamide mononucleotide solution for 25 days under a general condition.
FIG. 2 shows the comparative results of the stability of Example 1, Example 15, Example 16 and 0.5% aqueous nicotinamide mononucleotide solution for 25 days under an accelerated condition.
FIGs. 3(a)-(d) are photographs showing hair growth status respectively at day 0, 30, 60, and 120 of female test subjects who have used the hair serum containing nicotinamide mononucleotide of Example 4.
FIG. 4 illustrates the change of the average hair gap between hairlines of female test subjects who have used the hair serum containing nicotinamide mononucleotide of Example 4 as a function of days.

### DETAILED DESCRIPTION OF EMBODIMENTS

The following description of the specific embodiments further explains the present invention, but this is not a limitation to the invention. A person skilled in the art can make various modifications or improvements according to the basic concept of the invention, and they are within the scope of the invention as long as they do not depart from the basic concept of the invention.

The slow growth, gradual graying and loss of hair are the most obvious signs of aging, which can be caused by the lack of ATP supply to the hair follicle cells. The energy molecule ATP is synthesized in the mitochondria of the hair follicle cells through biochemical reactions such as glycolysis to provide enough energy for maintaining hair growth and normal functioning of the hair follicles. Studies have reported that hair follicle cells gradually die at low ATP levels, resulting in the cessation of hair growth, reduction of pigment cells and gradual graying of hair, and subsequent shedding and apoptosis. ATP synthesis in hair follicle cells is different from other cells. Generally, most cells in the body use glucose for this purpose and require less coenzyme I. Hair follicle cells are less likely to perform ATP synthesis by this method; instead, they mostly use glutamine as a substrate for ATP synthesis, and the amount of coenzyme I required for this synthesis pathway is multiplied. For numerous reasons, the level of coenzyme I in the body decreases each year with ageing, and the level of coenzyme I in the hair follicle cells decreases as well accordingly, such that ATP synthesis will become more difficult to meet the needs of normal cellular function. Hair follicle cells are also activated by lactic acid, which is synthesized by the reaction of pyruvic acid with the hydrogen ions provided in reduced coenzyme I, in the presence of lactate dehydrogenase which expresses lactic acid intracellularly as a catalytic enzyme. In the absence of lactic acid, the hair follicle cells cannot grow new hair. Thus, the simultaneous occurrence of both conditions is directly related to a decrease in the level of coenzyme I in the cells.

Coenzyme I is a biochemical substance that is essential for maintaining the health of hair follicles and promoting hair growth. Therefore, raising the level of coenzyme I in the body is the root solution to address the problems of slow hair growth, graying and hair loss. Although oral nicotinamide mononucleotide supplements can effectively raise the overall level of coenzyme I in the body, they can only provide limited supplementation of the active ingredients required by hair and hair follicle cells, and they cannot function immediately, so that the effect will gradually manifest itself only by taking the supplements for a long time.

To solve the above problems, the present invention provides a hair care composition comprising nicotinamide mononucleotide. The composition can be applied directly onto the hair, scalp, epidermis and hair follicles to directly replenish and enhance the level of coenzyme I in the hair follicles and epidermal cells in the most effective way, with the aim of enhancing ATP synthesis and lactic acid production, thereby maintaining hair follicle health and activating hair follicle cells to promote hair regrowth. The hair care composition can be used to alleviate, improve or even reverse various hair problems in middle-aged and elderly people, as well as to provide health benefits for hair health and to help growth of the hair.

The hair care compositions of the present invention are obtained by adding nicotinamide mononucleotide to a certain stabilizing base ingredient (e.g., certain surfactants, additives, antioxidants, etc., in a certain range of ingredient proportions). As the user applies the hair care product directly to the scalp, hair and skin, the main active functional ingredient, nicotinamide mononucleotide, is absorbed by the hair follicles and epidermal cells to synthesize coenzyme I with ATP in the mitochondria by nicotinamide mononucleotide adenylyltransferase. The coenzyme I participates in the biochemical synthesis of ATP and lactic acid in the hair follicles and epidermal cells. In addition, in the synthesis of lactic acid, the coenzyme I is converted into reduced coenzyme I which is the coenzyme required in the catalytic process of synthesis of lactic acid from pyruvic acid and serves as a medium for hydrogen ion transport. The hair care compositions of the present invention enhance the level of coenzyme I in hair follicles and epidermal cells to address the problems of graying and loss of hair due to aging, and can maintain hair health and accelerate the growth cycle of the hair.

Nicotinamide mononucleotide also maintains excellent liquid stability in the hair care compositions of the present invention, so that the hair care compositions can maintain its activity compared to conventional aqueous solutions of nicotinamide mononucleotide. The present invention promotes the applicability and commercializability of nicotinamide mononucleotide in hair care compositions, solves the long-standing problem in industrial production concerning the stability of nicotinamide mononucleotide in liquid state, and provides users with a root and novel solution, based on biochemical scientific researches, to their hair problems.

In one aspect, the present invention provides a hair care composition comprising nicotinamide mononucleotide or a salt thereof or a hydrate thereof, and a base ingredient for stabilizing the nicotinamide mononucleotide or the salt thereof or the hydrate thereof. The base ingredient for stabilizing the nicotinamide mononucleotide or the salt thereof or the hydrate thereof includes one or more of an anionic surfactant, an amphoteric surfactant, a cationic surfactant, a neutral surfactant, an antioxidant, a pH buffer, and an acidity regulator. The care compositions of the present invention are capable of maintaining the stability of nicotinamide mononucleotide or a salt thereof or a hydrate thereof in the aqueous care compositions over a long period of time, so that the hair care compositions containing nicotinamide mononucleotide or a salt thereof or a hydrate thereof can maintain their effective quality over a long period of time.

### Nicotinamide mononucleotide or a salt thereof or a hydrate thereof

In various embodiments of the present invention, the hair care composition comprises nicotinamide mononucleotide or a salt thereof or a hydrate thereof. The physical form of nicotinamide mononucleotide or a salt thereof or a hydrate thereof may be a solid, liquid or atomized form. The nicotinamide mononucleotide may have a chemical structure in acid or salt form, with crystalline water, or in hydrate, or other accessory form. The physical form and chemical structure may have a purity of 5% to 99.8%, preferably 80% to 99.8%, and more preferably 97.5% to 99.5%.

In specific embodiments of the present invention, the content of the nicotinamide mononucleotide or the salt thereof or the hydrate thereof ranges from 0.01 wt% to 25 wt%, preferably from 0.01 wt% to 20 wt%, and more preferably from 0.1 wt% to 15 wt%, based on the total weight of the hair care composition.

### Base ingredient

A feature of the present invention is that the hair care composition comprises a base ingredient for stabilizing nicotinamide mononucleotide or a salt thereof or a hydrate thereof. The base ingredient for stabilizing nicotinamide mononucleotide or a salt thereof or a hydrate thereof comprises one or more of an anionic surfactant, an amphoteric surfactant, a cationic surfactant, a neutral surfactant, an antioxidant, a pH buffer, and an acidity regulator.

### Anionic surfactant

In the hair care compositions of the present invention, the anionic surfactant is capable of stabilizing nicotinamide mononucleotide or a salt thereof or a hydrate thereof. The anionic surfactant may be a fatty alcohol polyether sulfate, and may also be a single one or a mixture of ammonium laureth sulfate, sodium laureth sulfate, sodium lauryl sulfate, ammonium lauryl sulfate, sodium methyl cocoyl taurate, laureth-5 carboxylic acid, and sodium lauryl glucose carboxylate.

In specific embodiments of the present invention, the content of the anionic surfactant is 15-65 wt%, preferably 25-45 wt%, and more preferably 30-40 wt%, based on the total weight of the hair care composition.

### Amphoteric surfactant

Amphoteric surfactants can exhibit both anionic and cationic properties and have excellent biocompatibility.

In the hair care compositions of the present invention, the amphoteric surfactant can be used not only as a detergent, but also to stabilize nicotinamide mononucleotide or a salt thereof or a hydrate thereof. The amphoteric surfactant may be an alkyl amino acid amphoteric surfactant or an alkyl amphoteric carboxylate. Amphoteric surfactants can include a single one or mixtures of cocamidopropyl betaine, cocamide monoethanolamine, lauryl glucoside, and coconut methyl dextran.

In specific embodiments of the present invention, the content of the amphoteric surfactant is 1-15 wt%, preferably 1.5-8 wt%, and more preferably 2-7 wt%, based on the total weight of the hair care composition.

Other amphoteric surfactants include alkyl amphoteric carboxylic acids or salts thereof, for example, alkali metal salts of alkyl amphoteric dipropionic acid, alkyl amphoteric diacetic acid, alkyl amphoteric glycine and alkyl amphoteric propionic acid, wherein said alkyl denotes an alkyl group having 6 to 20 carbon atoms. More preferably, the alkyl group is derived from coconut oil or a lauryl compound, such as coco amphoteric dipropionate salt. Such coco amphoteric dipropionate surfactants may be consistent with amphoteric surfactants of the same type and are sold under the trademark of Miranol series.

Commercially useful amphoteric surfactants include:
X coco amphoteric acetate (sold under the trademarks of MIRANOL ULTRA C-32 and MIRAPON FA);
X coco amphoteric propionate (sold under the trademarks of MIRANOL CMSF CONC. and MIRAPON FAS);
X coco amphoteric diacetate (sold under the trademarks of MIRANOL C2M CONC. and MIRAPON FB);
X lauroyl amphoteric acetate (sold under the trademarks of MIRANOL HM CONC. and MIRAPON LA);
X lauroyl amphoteric diacetate (sold under the trademarks of MIRANOL H2M CONC. and MIRAPON LB);
X lauroyl amphoteric dipropionate (sold under the trademarks of MIRANOL H2M-SF CONC. and MIRAPON LBS);
X lauroyl amphoteric diacetate obtained from a mixture of lauric and myristic acids (sold under the trademark of MIRANOL BM CONC.);
X coco amphoteric propyl sulfonate (sold under the trademark of Miranol CS CONC.);
X hexanoyl amphoteric diacetate (sold under the trademark of MIRANOL S2M CONC.);
× hexanoyl amphoteric acetate (sold under the trademark of MIRANOL SM CONC.);
× hexanoyl amphoteric dipropionate (sold under the trademark of MIRANOL S2M-SF CONC.); and
× stearoyl amphoteric acetate (sold under the trademark of MIRANOL DM).

The most preferred other amphoteric surfactant is coco amphoteric acetate.

Based on the total weight of the hair care composition, the other amphoteric surfactant comprises from about 0.1 wt% to about 7 wt%, preferably from about 0.2 wt% to about 4 wt%, and more preferably from 0.3 wt% to 3 wt%.

### Cationic Surfactant

The cationic surfactant can provide cations that impart functional utility to the hair care composition: the cations will attach to the hair during cleansing, which will straighten the hair, reduce the formation of hair tangles and make it easier to comb. At the same time, cations can interact with nicotinamide mononucleotide in the aqueous state to enhance its stability in the liquid state. Cationic surfactant may include amine salt heterocyclic quaternary ammonium salt type cationic surfactants. Specific examples of cationic surfactants include at least one of cetyl trimethyl ammonium chloride, dodecyl trimethyl ammonium chloride, tricetyl methyl ammonium chloride, stearyl dimethyl benzyl ammonium chloride, and di(partially hydrogenated tallow) dimethyl ammonium chloride, polyquaternium-10, guar hydroxypropyl trimethyl ammonium chloride, and preferably polyquaternium-10, guar hydroxypropyl trimethyl ammonium chloride, or polyethylene glycol.

In specific embodiments of the present invention, the cationic surfactant is present in an amount of 0.01-0.09 wt%, preferably 0.02-0.08 wt%, and more preferably 0.03-0.08 wt%, based on the total weight of the hair care composition.

In some embodiments of the present invention, the hair care composition may also comprise other surfactants, such as nonionic surfactants and other amphoteric surfactants.

The nonionic surfactants may act as emulsifiers and stabilizers in the compositions. The term "nonionic surfactant" as used herein includes mixtures of nonionic surfactants.

Examples of useful nonionic surfactants further include condensates of ethylene oxide and a hydrophobic portion, wherein the hydrophobic portion has an average hydrophilic-lipophilic balance (HLB) of between about 8 and about 16, and more preferably between about 10 and about 12.5. These surfactants comprise condensation products of an aliphatic primary or secondary alcohol having a linear or branched chain configuration of from about 8 to about 24 carbon atoms and ethylene oxide, wherein the ethylene oxide is from about 2 moles to about 40 moles, and preferably from about 2 moles to about 9 moles, relative to each mole of alcohol.

In a preferred embodiment, the aliphatic alcohol comprises from about 9 to about 18 carbon atoms and is ethoxylated with from about 3 moles to about 12 moles of ethylene oxide per mole of aliphatic alcohol. Particularly preferred is the ethoxylation of a primary alcohol having from about 12 to about 15 carbon atoms with from about 5 moles to about 9 moles ethylene oxide per mole of the alcohol. One of such materials is marketed by Shell Chemical Company under the trade name Neodol 25-9. Other commercially available nonionic surfactants are marketed by Shell Chemical Company as Neodol 25-6.5 and Neodol 25-7.

Other suitable nonionic surfactants include condensation products of alkyl phenols having from about 6 to about 12 carbon atoms and ethylene oxide ranging from about 3 moles to about 30 moles, and preferably from about 5 moles to about 14 moles. Examples of such surfactants are marketed by Rhodia, Inc. under the trade names of IGEPAL CO 530, IGEPAL CO 630, IGEPAL CO 720 and IGEPAL CO 730. The most preferred nonionic surfactants are ethoxylates of the mixed linear alcohols, such as Laureth-7 sold by Rhodia, Inc. under the trade name RHODASURF L-790.

### Antioxidant

Antioxidants are effective in preventing oxidation of the substances, thereby avoiding the formation of oxidation products such as free radicals during the oxidation and subsequent unpredictable chemical reactions with the main components that would cause deterioration and degradation of the main components over time. Nicotinamide mononucleotide would be oxidized and degraded into nicotinamide and other substances in the liquid state, and therefore, the use of antioxidants can effectively protect the stability of nicotinamide mononucleotides in liquid form. Antioxidants can be divided into two categories: synthetic antioxidants and natural antioxidants. These two types of antioxidants behave differently in protecting the chemical structure of nicotinamide mononucleotide in liquid form, and natural antioxidants are preferred. The natural antioxidants can be further subdivided into amino acid antioxidants and vitamin antioxidants, preferably a single one or mixtures of L-cysteine, DL-methionine, vitamin A, vitamin C, vitamin E, vitamin P and sodium phytate. In addition to the inherent efficacy, all of the mentioned antioxidants also exert a degree of preservative effect, which contributes synergistically to the overall stability of the hair care composition.

In specific embodiments of the present invention, the content of the antioxidant is 0.5-3 wt%, preferably 0.9-2.5 wt%, and more preferably 1.5-2 wt%, based on the total weight of the hair care composition.

### Acidity regulator

The stability of nicotinamide mononucleotide in the liquid state is increased in a weakly acidic environment, and therefore, pH regulation using acidity regulators is required in the hair care compositions. The acidity regulator of the hair care composition may be a single one of citric acid, oxalic acid, lactic acid and the like, or a mixture thereof.

In specific embodiments of the present invention, the content of the acidity regulator is 1-3 wt%, preferably 1.5-2.5 wt%, and more preferably 2-2.5 wt%, based on the total weight of the hair care composition.

### Stabilizer

The nicotinamide mononucleotide or salt thereof or hydrate thereof is usually very unstable in the compositions and are prone to degradation. Therefore, the hair care compositions of the present invention also comprise additives for stabilizing the activity of the nicotinamide mononucleotide or salt thereof or hydrate thereof.

The stabilizer may be one or more selected from sialic acid, theanine, beta-carotene and sodium phosphate.

### Other ingredients

Other different ingredients may be added to the hair care composition to enrich its appearance, texture, fragrance, functional properties and user experience, provided that the ingredient or combination thereof does not undergo any chemical reaction with the nicotinamide mononucleotide that would result in significant degradation of the nicotinamide mononucleotide during its shelf life. At the same time, the ingredients may improve the stability of nicotinamide mononucleotide or a salt thereof or a hydrate thereof in the liquid to some extent.

In terms of appearance and texture, a conditioning agent (e.g., propylene glycol) can be added to the hair care composition, rendering it transparent, opaque or colored in appearance and aqueous to varying degrees of viscosity in texture. Propylene glycol can be used to increase the liquid viscosity of the hair care composition, and the manufacturer can adjust the viscosity according to the positioning of the product thereof so that increasing the amount of propylene glycol in the hair care composition can increase the viscosity proportionally. In specific embodiments of the present invention, the content of the other ingredients (e.g., propylene glycol) is 0.01-1 wt%, preferably 0.1-0.8 wt%, and more preferably 0.2-0.6 wt%, based on the total weight of the hair care composition.

A co-solvent may also be added to the hair care composition to adjust the physical texture in the composition, in particular the translucency in the liquid appearance of the hair care composition. Sodium xylenesulfonate can provide increased clarity in the liquid form of the hair care product such as shampoo, conditioner and serum. Sodium xylenesulfonate can increase the solubility of the water molecules in the liquid to the ingredients, allowing for more sufficient contact among the various ingredients in the hair care composition. The nicotinamide mononucleotide of the hair care composition has a higher solubility in the hair care composition, and specific products such as highly concentrated nicotinamide mononucleotide serum can be made to meet different needs and expectations of users. Furthermore, sodium xylenesulfonate allows the fusion of nicotinamide mononucleotide with other ingredients so that some of the products in the hair care compositions can provide more than one functional effect at the same time, which can be understood as the use of nicotinamide mononucleotide or its derivatives in the hair care composition to promote the level of coenzyme I in various tissues of the head, including the hair, scalp, hair follicles and cells thereof, to achieve improved hair and head health, and in addition thereto, other purposes such as cleansing, styling coordination, odor modification, etc., may also be achieved at the same time, which should be considered as part of the functionality of the hair care composition. In specific embodiments of the present invention, the content of sodium xylenesulfonate is 0.01-10 wt%, preferably 0.1-5 wt%, and more preferably 1-3 wt%, based on the total weight of the hair care composition.

A foaming agent can also be added to the hair care composition to increase the cleaning and repairing function of some monofunctional or multifunctional products in the composition, such as shampoo and conditioner. The foam can create a larger contact space, which allows the main ingredient and other ingredients in the hair care composition to spread effectively on the head, thus creating favorable conditions for the hair, scalp, hair follicles, etc., to absorb the main ingredient, nicotinamide mononucleotide or its derivatives, into the cells for the purpose of enhancing the level of coenzyme I, especially in the mitochondria of the cells, to cope with daily metabolism and biocatalysis. Nicotinamide mononucleotide or its derivative also protects against and repairs the damage caused by environmental pollution and UV exposure.

The foam also prolongs the residence time of the ingredients on the head. The foam bubbles can sustain each other by the tension among them, such that they can remain easily between the hair and the scalp, while not being affected by differences in the shape of the head. Therefore, the addition of foaming agents to the hair care compositions also has a physical effect in enhancing the coenzyme I. The foaming agent that can be used in the hair care composition may be a quaternary ammonium polyethylene glycol alkyl amino acid surfactant, which may be betaine, cocamidopropyl betaine, cocoyl monoethanolamine, sodium cocoyl glycinate, sodium lauryl glucose carboxylate, sodium salt of olive oil fatty acid polyethylene glycol ester mono(carboxymethyl) ether, etc. The above-mentioned foaming agents can be added to the ingredients of the hair care composition according to the function of the product, and these foaming agents can be used alone or as a mixture in various proportions in the formulation of the product, in order to optimize the foam condition of the product to achieve the best performance and user experience. As a component of the hair care composition, the foaming agent is preferably betaine, sodium cocoyl glycinate and sodium lauryl glucose carboxylate, and more preferably sodium cocoyl glycinate. In specific embodiments of the present invention, the content of sodium cocoyl glycinate is 0.01-10 wt%, preferably 0.1-5 wt%, and more preferably 0.5-1 wt%, based on the total weight of the hair care composition. As a mixture in various proportions, the combination of sodium cocoyl glycinate and sodium lauryl glucose carboxylate is the most preferred one. The ratio of sodium cocoyl glycinate to sodium lauryl glucose carboxylate is 2:1 for fine foams or 1:2 for larger foams. The size of the foams can be adjusted proportionally by the combination of sodium cocoyl glycinate and sodium lauryl glucose carboxylate in the mixture. In specific embodiments of the present invention, the content of the total mixture is 0.01-10 wt%, preferably 0.1-5 wt%, and more preferably 0.5-1 wt%, based on the total weight of the hair care composition.

Other additives may also be added to the hair care composition to enrich its appearance, aroma and use feeling and function. In terms of appearance, conventional edible pigments may be added to the hair care composition. Those with darker colors such as yellow, brown, blue and black will have a more pronounced performance in light avoidance, and also will have a certain effect on the stability of the main and other components of the hair care composition. In specific embodiments of the present invention, the content of edible pigments is 0.01-1 wt%, preferably 0.05-0.1 wt%, and more preferably 0.05-0.08 wt%, based on the total weight of the hair care composition. Fragrance is more important for such products of the hair care composition as shampoos, conditioners and serums, and can increase the degree of user satisfaction with the product, while addressing the smell of odor in the hair. The hair care composition may include non-hazardous fragrances or refined plant- or mineral-based fragrant oils as the primary source of fragrance, more preferably refined plant- or mineral-based fragrant oils. Since the formulation of the hair care composition doesn't have any odor, the fragrance provided by the additive should be indeed reflected in the hair care composition In other words, the fragrance of the composition comprising the additive should not have any difference from the inherent fragrance of the additive. Otherwise, unexpected chemical reaction seems to have occurred between the additive and the hair care composition, which may have an effect on the chemical structure of the nicotinamide mononucleotide in the hair care composition, thereby altering the function of the hair care composition and stability thereof. The refined plant- or mineral-based fragrant oils are mostly inert and would not cause the above-mentioned particular condition when added to the hair care composition. In specific embodiments of the present invention, the content of the refined plant- or mineral-based fragrant oils is 0.001-0.1 wt%, preferably 0.005-0.01 wt%, and more preferably 0.08-0.01 wt%, based on the total weight of the hair care composition.

The hair care composition may incorporate functional additives to meet the needs of different users, which can collaboratively enhance the level of coenzyme I in the hair, scalp and hair follicle cells, and prompt the hair care composition to exhibit significant effects over a short period of usage in line with the user's expectations. When the epidermal cells of the head have decreased level of coenzyme I, it is difficult to synthesize ATP to cope with the high energy demand of daily life. Apoptosis of epidermal cells leads to an increase in scalp, and at the same time, the immunity of the cells is reduced and the chance of infection is increased. Scalp hoarding not only affects the social appearance, but also it makes difficult to drain sweat and oil from the epidermis of the headfor dust to attach to the head because of the increased surface area. With both the decline of physiological health and the deterioration of the head, the inflammation of the epidermis of the head tends to occur frequently, which is known in the art as intense head itch. The itchiness of the head forces the patient to touch it with his hands, and when the itchiness increases, the patient strokes the scalp frequently or even scratches it vigorously to remove the unpleasant sensation in the shortest possible time. In the process, the patient causes damage to the epidermis of his head, especially to the hair follicles, which can cause hair loss and the dead follicle cells to lose their ability to grow hair. These physical injuries are partially permanent, and even raising the level of coenzyme I cannot revital the cells's function. The functional additives may be pain or itching relievers and scalp inhibitors, among which use of peppermint oil and zinc pyrithione as the functional additives of the hair care composition can serve the purpose. The peppermint oil gives the user a cooling sensation and interferes with the unpleasant feeling of itchiness, when it is brought into contact with the epidermis of the head. Thus, the user can avoid scratching his head due to itchiness and hurting the tissues and cells in the process. In specific embodiments of the present invention, the content of peppermint oil is 0.05-0.1 wt%, preferably 0.05-0.09 wt%, and more preferably 0.06-0.08 wt%, based on the total weight of the hair care composition. Zinc pyrithione is an antimicrobial agent that inhibits the growth of gram-negative and positive bacteria as well as mould and is commonly used as a dandruff removal component with good efficacy. In specific embodiments of the present invention, the content of zinc pyrithione is 0.1-1.5 wt%, preferably 0.5-1 wt%, and more preferably 0.8-1 wt%, based on the total weight of the hair care composition.

In some embodiments of the present invention, the hair care product form of the hair care composition is at least one selected from shampoo, conditioner, serum, soap, spray, cream, hair wax, gel, paste, or any liquid or solid. Thus, the hair care products of the present invention may also have other functions such as cleansing, conditioning, anti-dandruff and fixing hair style. For example, the shampoo containing nicotinamide mononucleotide also has a cleansing effect, i.e., generally for removal of oil and odor from the hair; and the shampoo is also foamy in order to penetrate easily into the hair and scalp.

In specific embodiments of the present invention, the shampoo may contain water, nicotinamide mononucleotide or a salt thereof or a hydrate thereof, a surfactant, an acidity regulator, a fragrance or the like as excipients to preserve the function, properties and durability of the shampoo. In more specific embodiments, the content of water is from 30 wt% to 90 wt% based on the total weight of the shampoo.

The hair care composition includes, in addition to the hair product, an eyebrow care product, an eyelash care product and a beard care product. The eyebrow care product mainly includes eyebrow care lotion and eyebrow care cream. The eyebrow care lotion and the eyebrow care cream contain 0.01-3% (w/v) of nicotinamide mononucleotide or a salt thereof or a hydrate thereof as the main ingredient, with the addition of excipients including sage oil, green tea extract, white beeswax, etc. The eyelash care product is an eyelash care solution assembled with an eyelash brush for applying the eyelash solution to the parts of the eyelashes, including both the upper and lower eyelash parts and the hair follicle parts thereof. The main ingredient of the eyelash lotion is also nicotinamide mononucleotide or a salt thereof or a hydrate thereof. Since eyelashes grow more slowly than hair, more of the main ingredient is needed to stimulate their growth cycle, such that the eyelash lotion contains 1-10% (w/v) of nicotinamide mononucleotide or a salt thereof or a hydrate thereof with tea tree extract and cysteine as excipients. Since the eyelash lotion is used near eyes, the composition thereof should not contain materials that would irritate the eyes, volatile substances, or materials for treating eye diseases. The eyelash care lotion is assembled to give the users who concern about the health of their eyelashes, especially those with slow-growing and short eyelashes, an option to improve the condition of their eyelashes and help them with both eye health care and personal grooming. The beard care products are beard care liquid, beard care spray and beard care cream. The main ingredient of the beard care products is 0.1-2% nicotinamide mononucleotide or a salt thereof or a hydrate thereof, and the excipients include mint extract, burdock powder or its extract, vitamin A, vitamin E, cysteine, etc. The products are available in liquid, spray and cream forms comprising water, ethanol at a low concentration of 0.01-0.05% (v/v) or beeswax as a medium, to suit the user's preference. The beard care liquid, beard care spray and beard care cream are intended to accelerate the growth of the beard, maintain or restore the original color and the health of the hair follicles in the same principle as described above with regard to the hair care compositions, thus providing the users with a scientifically healthy product for personal styling.

In another aspect, the invention also provides a method for preparing a hair care composition, comprising the steps of: providing nicotinamide mononucleotide or a salt thereof or a hydrate thereof; providing an aqueous solution of a base ingredient for stabilizing the nicotinamide mononucleotide or the salt thereof or the hydrate thereof; and mixing the nicotinamide mononucleotide or the salt thereof or the hydrate thereof with the aqueous solution and adjusting the pH to obtain the hair care composition. The base ingredient for stabilizing the nicotinamide mononucleotide or the salt thereof or the hydrate thereof comprises one or more of an anionic surfactant, an amphoteric surfactant, a cationic surfactant, an antioxidant, a pH buffer and an acidity regulator.

Preferably, the method comprises:
(1) blending one or more fatty alcohol polyether sulfate anionic surfactants to obtain a first type of surfactant;
(2) blending one or more alkyl acyl amino acid amphoteric surfactants to obtain a second type of surfactant;
(3) blending one or more cationic surfactants to obtain a third type of surfactant;
(4) blending the first type of surfactant, the second type of surfactant and the third type of surfactant to obtain a complex surfactant;
(5) adding nicotinamide mononucleotide to the complex surfactant, thereby obtaining a mixture; and
(6) adding an amino acid-based antioxidant to the mixture and adjusting the pH of the mixture by an acidity regulator to a pH value in the range of 4.85-7.0, thereby obtaining the hair care composition.

In more specific embodiments, the surfactants may be ammonium laureth sulfate, cocoyl glutamate TEA salt and MIRANOL. Based on the total weight of the shampoo, ammonium laureth sulfate is present in an amount of 0.2 wt% to 1.9 wt%, cocoyl glutamate TEA salt is present in an amount of 0.1 wt% to 1.5 wt%, and MIRANOL is present in an amount of 0.3 wt% to 3 wt%. In more specific embodiments, the acidity regulator is potassium dihydrogen phosphate and the pH of the shampoo is 4.5 to 5.5. In more specific embodiments, the fragrance may be organic incense oils with different aromas selected to match the market needs and present in an amount of 0.1 wt% to 0.5 wt%. In a further specific embodiment, the fragrance may be different organic incense oils with different aromas selected to match the market needs and present in an amount of 0.1 wt% to 0.5 wt%. In more specific embodiments, the shampoo may also contain shading agents, preservatives, etc. The content of these ingredients should not exceed 0.05 wt%, based on the weight of the shampoo.

In specific embodiments of the present invention, the serum is mainly used to elevate the level of coenzyme I and reduced coenzyme I in the hair follicle cells, and soothe itchy or inflamed scalp conditions, thereby promoting the health of the hair follicle stem cells and activating their activity to synthesize ATP and lactic acid. Thus, the main component of the serum is nicotinamide mononucleotide or a salt thereof or a hydrate thereof which is present in an amount of 3 wt% to 25 wt%, preferably 5 wt% to 15 wt%, and more preferably 8 wt% to 12 wt%, based on the total weight of the composition. In more specific embodiments, the components of the serum are green tea extract, peppermint oil, sage oil, burdock powder, beeswax, and water, among others. In addition, acetyl glutathione and glutathione may be added to the serum.

Further, as long as the hair care composition is a hair product for external use, the addition of nicotinamide mononucleotide to the product, or the use of the product for elevating the level of coenzyme I in hair follicles and epidermal cells to achieve prevention, repair, improvement or reversal of aging of the hairs, should be considered as falling within the scope of this patent application.

The preparation of the hair care compositions according to the present invention is described below, by taking shampoo and serum as examples.

The shampoo is prepared as follows. Ammonium laureth sulfate, cocoyl glutamate TEA salt and MIRANOL are added to a container to serve as surfactants. The contents of these three surfactants can be different or equal, mainly depending on different hair types, and the hair is neutral when the contents thereof are equal. In the case of the neutral hair, the total content of the three surfactants ranges from 0.1 wt% to 10 wt%, preferably from 0.5 wt% to 10 wt%, and more preferably from 1 wt% to 10 wt%, based on the total weight of the shampoo. After adding the surfactants and vitamins, pure water is added to 80 wt% of the total weight, followed by an acidity regulator. The acidity regulator may be potassium dihydrogen phosphate or other buffers, and the pH of the solution is preferably in the range of from 4.8 to 6, and more preferably from 5 to 5.5. After adjusting the pH to 4.5-5.5, nicotinamide mononucleotide or a salt thereof or a hydrate thereof may be added. Based on the total weight of the shampoo, the content of nicotinamide mononucleotide or a salt thereof or a hydrate thereof is 0.01 wt% to 25 wt%, preferably 0.1 wt% to 5 wt%, and more preferably 0.1 wt% to 1 wt%. Then, organic incense oils, shading agents, preservatives and the like can be added, and the total content of these ingredients does not exceed 5% of the total weight of the shampoo. The mixture is stirred evenly and then pure water is added up to the total amount, such that the shampoo is prepared and ready for use.

The user can apply the shampoo evenly onto head in an amount depending on personal preference, and it is recommended that massage be performed on head for 2-5 minutes, before the shampoo is rinsed off. The shampoo should be used at least once a day and should be used continuously to achieve the target effect.

The preparation of the serum is as follows. First, burdock powder, green tea extract and nicotinamide mononucleotide are added to a container; wherein, based on the total weight of the serum, the content of burdock powder is in the range of from 1 wt% to 5 wt%, preferably from 1 wt% to 4 wt%, and more preferably from 1 wt% to 2.5 wt%; the content of green tea extract is in the range of from 1 wt% to 5 wt%, preferably from 2 wt% to 4 wt%, and more preferably from 2 wt% to 2.5 wt%; the content of the nicotinamide mononucleotide is in the range of from 3 wt% to 25 wt%, preferably 5 wt% to 15 wt%, and more preferably 8 wt% to 12 wt%. Auxiliary materials such as sialic acid, theanine, glycyrrhizic acid, glutathione and S-acetyl-L-glutathione are added, too. Then, after adding pure water to 80% of the total weight, potassium dihydrogen phosphate is added as an acidity regulator to adjust a pH of 5 to 5.2, and the mixture is stirred homogeneously. To the resultant solution, a peppermint oil and sage oil are added separately; wherein, based on the total weight of the serum, the content of peppermint oil is 0.1 wt% to 1.5 wt%, preferably 0.1 wt% to 1.2 wt%, and more preferably 0.1 wt% to 1 wt%, and the content of sage oil is 0.1 wt% to 1.5 wt%, preferably 0.1 wt% to 1.2 wt%, and more preferably 0.1 wt% to 1 wt%. Finally, white beeswax, which has been hot-melted at 62 to 65 degrees Celsius, is added in an amount of 0.1% of the total weight; and pure water is added to the predetermined weight and the mixture is stirred until completely dissolved.

After washing hair, the user may apply 5-10 ml of the serum directly to the scalp and the hair root, gently press the head with hands to spread the serum evenly on the scalp until the serum is completely absorbed. The serum should be used at least once a day and should be used continuously to achieve the target effect.

The content of the invention is further explained or illustrated below by way of examples, but these embodiments and examples should not be construed as limiting the scope of protection of the invention.

### Examples

Where specific conditions are not indicated in the following examples, they are carried out under conventional conditions or those recommended by the manufacturers.

The materials and equipment used in the examples are described as follows.
Nicotinamide mononucleotide: obtained from GeneHarbor (Hong Kong) Biotechnologies Ltd.
Ammonium laureth sulfate: obtained from Jining Baichuan Chemical Co., Ltd.
Cocoyl glutamate TEA salt: obtained from Beijing Lingbao Technology Co., Ltd.
MIRANOL: obtained from Suzhou Yuantairun Chemical Co., Ltd.
Burdock powder: obtained from Xinghua Hongsheng Food Co., Ltd.
Green tea extract: obtained from Shaanxi Hengling Natural Biological Products Co., Ltd.
Peppermint oil: obtained from Jiangxi Jiping Natural Vegetable Oil Co., Ltd.
Sage oil: obtained from Shenzhen Austin Trading Co., Ltd.
Potassium dihydrogen phosphate: obtained from Jiangsu Zidong Food Co., Ltd.
White beeswax: obtained from Guangzhou Riyou Technology Co., Ltd.
S-acetyl-L-glutathione: obtained from GeneHarbor (Hong Kong) Biotechnologies Ltd.
Glutathione: obtained from GeneHarbor (Hong Kong) Biotechnologies Ltd.
Theanine: obtained from Merck, USA;
Sialic acid: obtained from Merck, USA;
Glycyrrhizic acid: obtained from Merck, USA;
Small stirrer (LIAEBO LSJB120): obtained from Changzhou, Jiangsu;
pH meter (LEICI PHB-4): from Shanghai, China

### Example 1: Nicotinamide mononucleotide-containing shampoo for neutral hair

500 ml of nicotinamide mononucleotide-containing shampoo for neutral hair was prepared as follows. 250 ml of water, 25 g of ammonium laureth sulfate, 25 g of cocoyl glutamate TEA salt, 1 g of MIRANOL, and 50 g of vitamin C were added, and then pure water was added to a total of 400 ml with stirring by a small stirrer at about 100 rpm for 15 minutes until completely homogeneous. The pH of the solution was measured using a pH meter (LEICI PHB-4, Shanghai), and the pH value was adjusted to 5.5 using potassium dihydrogen phosphate. Then, 5 g of nicotinamide mononucleotide was added, and pure water was added to a total amount of 500 ml with stirring until completely homogeneous.

### Example 2: Nicotinamide mononucleotide-containing shampoo for dry hair

500 ml of nicotinamide mononucleotide-containing shampoo for dry hair was prepared as follows. 250 ml of water, 40 g of ammonium laureth sulfate, 10 g of cocoyl glutamate TEA salt, and 50 g of vitamin C were added, and then pure water was added to a total of 400 ml with stirring by a small stirrer at about 100 rpm for 15 minutes until completely homogeneous. The pH of the solution was measured with a pH meter (LEICI PHB-4, Shanghai) and adjusted with potassium dihydrogen phosphate to pH 5.5. Then, 5 g of nicotinamide mononucleotide was added, and pure water was added to a total of 500 ml with stirring until completely homogeneous.

### Example 3: Nicotinamide mononucleotide-containing shampoo for oily hair

500 ml of nicotinamide mononucleotide-containing shampoo for oily hair was prepared as follows. 250 ml of water, 10 g of ammonium laureth sulfate, 40 g of cocoyl glutamate TEA salt, and 50 g of vitamin C were added, and then pure water was added to a total of 400 ml with stirring by a small stirrer at about 100 rpm for 15 minutes until completely homogeneous. The pH of the solution was measured with a pH meter (LEICI PHB-4, Shanghai) and adjusted with potassium dihydrogen phosphate to 5.5. Then, 5 g of nicotinamide mononucleotide was added, and pure water was added to a total of 500 ml with stirring until completely homogeneous.

### Example 4: Hair serum containing nicotinamide mononucleotide

50 ml of hair serum containing nicotinamide mononucleotide was prepared as follows. 25 ml of water and 0.5 g of burdock powder, 0.4 g of sialic acid, 0.2 g of glutathione, 0.05 g of S-acetyl-L-glutathione, 0.1 g of theanine, 0.2 g of glycyrrhizic acid, 0.5 g of green tea extract, 5 g of vitamin C and 5 g of nicotinamide mononucleotide were added in that order. Subsequently, pure water was added to a total of 35 ml with stirring by a small stirrer at about 100 rpm for 15 minutes until completely homogeneous, and 0.5 g of ammonium laureth sulfate was added. The pH of the solution was measured using a pH meter and adjusted to pH 5.5 using potassium dihydrogen phosphate. Then, 500 ul of peppermint oil and 500 ul of sage oil were added, and a total of 0.1 g white beeswax which had been heated at 62-65 degrees Celsius was added in five portions with stirring at 250 rpm for 15 minutes until complete dissolution.

### Example 5: Anti-itch shampoo containing nicotinamide mononucleotide

500ml of nicotinamide mononucleotide-containing anti-itch shampoo was prepared as follows. 250 ml of water, 25 g of ammonium laureth sulfate, 25 g of cocoyl glutamate TEA salt, and 50 g of vitamin C were added, and then pure water was added to a total of 400 ml with stirring by a small stirrer at about 100 rpm for 15 minutes until completely homogeneous. The pH of the solution was measured with a pH meter and adjusted to pH 5.5 with potassium dihydrogen phosphate. Then, 2.5 g of nicotinamide mononucleotide and 2.5 ml of peppermint oil were added with stirring at about 100 rpm for 15 minutes until completely homogeneous, and pure water was added to a total of 500 ml with stirring until completely homogeneous.

### Example 6: Anti-dandruff functional shampoo containing nicotinamide mononucleotide

500 ml of nicotinamide mononucleotide-containing anti-dandruff shampoo was prepared as follows. 250 ml of water, 25 g of ammonium laureth sulfate, 25 g of cocoyl glutamate TEA salt, 50 g of vitamin C and 5 g of zinc pyrithione were added; and then pure water was added to a total of 400 ml with stirring by a small stirrer at about 100 rpm for 15 minutes until completely homogeneous. The pH of the solution was measured using a pH meter and adjusted to pH 5.5 using potassium dihydrogen phosphate. Then, 2.5 g of nicotinamide mononucleotide was added, and pure water was added to a total of 500 ml with stirring until completely homogeneous.

### Example 7: Multifunctional shampoo containing nicotinamide mononucleotide

Multifunctional shampoo containing nicotinamide mononucleotide is a product that has both anti-itch and anti-dandruff functions. 500 ml of nicotinamide mononucleotide -containing multifunctional shampoo was prepared as follows. 250 ml of water, 25 g of ammonium laureth sulfate, 25 g of cocoyl glutamate TEA salt, 50 g of vitamin C and 5 g of zinc pyrithione were added, and then pure water was added to a total of 400 ml with stirring by a small stirrer at about 100 rpm for 15 minutes until completely homogeneous. The pH of the solution was measured with a pH meter and adjusted to pH 5.5 with potassium dihydrogen phosphate. Then, 2.5 g of nicotinamide mononucleotide and 2.5 ml of peppermint oil were added with stirring at about 100 rpm for 15 minutes until completely homogeneous, and pure water was added to a total of 500 ml with stirring until completely homogeneous.

### Example 8: Eyebrow care liquid containing nicotinamide mononucleotide

50 ml of eyebrow care liquid containing nicotinamide mononucleotide was prepared by a process of firstly adding 0.5 g of nicotinamide mononucleotide, followed by adding 0.5 g of green tea extract and 20 ml of pure water with stirring until complete dissolution. The following ingredients were added to the solution under continuous stirring, in the order and amounts described below: 1. 0.25 g of cysteamine hydrochloride; 2. 0.25 g of vitamin C; 3. 0.1 g of vitamin E; 4. 0.5 ml of sage oil; 5. 0.05g of sodium citrate; 6. 0.1 g of citric acid; 7. 0.05 g of cocoyl amino acid salt. The mixture was stirred until completely dissolved, and then pure water was added to 50 ml, and the pH value was measured by a pH meter. The pH of the eyebrow care liquid should be between 4.75 and 5, which could be adjusted by using 1M sodium citrate solution or 1M citric acid solution. The eyebrow care liquid was used in the morning and at night by the following method: gently applying about 1 ml of the liquid on a fingertip to the eyebrow region and gently swiping it back and forth until completely absorbed by the eyebrow region.

### Example 9: Eyebrow care cream containing nicotinamide mononucleotide

50 g of eyebrow care cream containing nicotinamide mononucleotide was prepared by a process of firstly adding 0.5 g of nicotinamide mononucleotide, followed by adding 0.5 g of green tea extract and 20ml of pure water with stirring until complete dissolution. The following materials were added to the solution under continuous stirring, in the order and amounts described below: 1. 0.25 g of cysteamine hydrochloride; 2. 0.25 g of vitamin C; 3. 0.1 g of vitamin E; 4. 0.5 ml of sage oil; 5. 0.05 g of sodium citrate; 6. 0.1 g of citric acid; 7. 0.05 g of cocoyl amino acid salt. The mixture was stirred until completely dissolved, and then pure water was added to 45 ml, and the pH value was measured by a pH meter. The pH value of the solution should be between 4.75 and 5, which could be adjusted using 1M sodium citrate solution or 1M citric acid solution. Afterwards, a total of 2 g white beeswax which had been heated at 62-65 degrees Celsius was added in gradually reduced batches with stirring, and the solution would gradually change from liquid into solid and paste-like. The eyebrow care cream was used in the morning and at night by the following method: gently applying a small amount of the cream on a fingertip to the eyebrow region and gently swiping it back and forth until completely absorbed by the eyebrow region.

### Example 10: Eyelash care kit containing nicotinamide mononucleotide

50 ml of eyelash care liquid containing nicotinamide mononucleotide was prepared by a process of firstly adding 5 g of nicotinamide mononucleotide, 1 g of tea tree extract, 1 g of green tea extract, 0.5 g of L-cysteine hydrochloride, 0.5 g of vitamin C and 0.1 g of vitamin D, followed by adding 40 ml of pure water with stirring until complete dissolution. The pH value of the liquid was measured with a pH meter and adjusted to pH 6.75-7 with 0.5 M NaOH, followed by filtering through a 0.22 uM filter membrane. In use, about 1-2 ml of the eyelash care liquid attached to an eyelash brush was gently swept along the eyelash region until completely absorbed. It is recommended that the eyelash care liquid be used in the morning and at night.

### Example 11: Beard care liquid containing nicotinamide mononucleotide

50 ml of the beard care liquid was prepared by a process of firstly adding 0.5 g of nicotinamide mononucleotide, 0.1 g of burdock powder, 0.08 g of vitamin A, 0.05 g of vitamin E, and 0.1 g of L-cysteine hydrochloride, followed by adding 40 ml of pure water with stirring until complete dissolution. The liquid was measured with a pH meter, and the pH value thereof was adjusted with 1 M NaOH to 5.25-5.5. Upon the adjustment, 0.01 g of glycerol cocoate and 0.05 ml of anhydrous ethanol were added with stirring until completely dissolved. The care liquid may be used in the morning and at night by the . following method: applying 1-2 ml of the care liquid to the beard growth region, and gently massaging the same until it is completely absorbed by the skin.

### Example 12: Beard care cream containing nicotinamide mononucleotide

50 g of the beard care cream was prepared by a process of firstly adding 0.5 g of nicotinamide mononucleotide, 0.1 g of burdock powder, 0.08 g of vitamin A, 0.05 g of vitamin E, and 0.1 g of L-cysteine hydrochloride, followed by adding 40 ml of pure water with stirring until complete dissolution. The solution was measured using a pH meter and the pH thereof was adjusted to 5.25-5.5 with 1M NaOH. After the adjustment, 0.01 g of glycerin cocoate was added with stirring until completely dissolved. Then, 1 g of white beeswax which had been preheated at 60-65 degrees Celsius was added in gradually reduced batches with continuous stirring, and the solution would gradually change into solid and paste-like. The cream may be used in the morning and at night by the following method: applying a small amount of the cream to the beard growth region and repeatedly massaging until it is absorbed by the skin.

### Example 13: Shampoo prototype (without the addition of nicotinamide mononucleotide)

500 ml shampoo prototype was prepared as follows. 250 ml of water, 25 g of ammonium laureth sulfate, 25 g of cocoyl glutamate TEA salt and 50 g of vitamin C were added, and then pure water was added to a total of 400 ml with stirring by a small stirrer at about 100 rpm for 15 minutes until completely homogeneous. The pH of the solution was measured using a pH meter and adjusted to pH 5.5 using potassium dihydrogen phosphate, and pure water was added to a total of 500 ml with stirring until completely homogeneous.

### Example 14: Hair serum prototype (without the addition of nicotinamide mononucleotide)

50 ml of hair serum without containing nicotinamide mononucleotide was prepared as follows. 25 ml of water and 0.5 g of burdock powder, 0.4 g of sialic acid, 0.2 g of glutathione, 0.05 g of S-acetyl-L-glutathione, 0.1 g of theanine, 0.2 g of glycyrrhizic acid, 0.5 g of green tea extract, and 5 g of vitamin C were added in that order. Subsequently, pure water was added to a total of 35 ml with stirring by a small stirrer at about 100 rpm for 15 minutes until completely homogeneous, and 0.5 g of ammonium laureth sulfate was added. The pH of the solution was measured using a pH meter and adjusted to pH 5.5 using potassium dihydrogen phosphate. Then, 500 ul of peppermint oil and 500 ul of sage oil were added, and a total of 0.1 g white beeswax which had been heated at 62-65 degrees Celsius was added in five batches with stirring at 250 rpm for 15 minutes until complete dissolution.

### Example 15: (Comparative Example, with the addition of antioxidant)

500 ml of the solution of Example 5 was prepared as follows. 250 ml of water and 50 g of vitamin C were added; and then pure water was added to a total of 400 ml with stirring by a small stirrer at about 100 rpm for 15 minutes until completely homogeneous. The pH of the solution was measured using a pH meter and adjusted to pH 5.5 using potassium dihydrogen phosphate and dipotassium hydrogen phosphate. Then, 5 g of nicotinamide mononucleotide was added, and pure water was added to a total amount of 500 ml with stirring until completely homogeneous.

### Example 16: (Comparative Example, with the addition of surfactants but at a low dose)

500 ml of shampoo prototype was prepared as follows. 250 ml of water, 0.25 g of ammonium laureth sulfate, 0.25 g of cocoyl glutamate TEA salt and 50 g of vitamin C were added, and then pure water was added to a total of 400 ml with stirring by a small stirrer at about 100 rpm for 15 minutes until completely homogeneous. The pH of the solution was measured using a pH meter and adjusted to pH 5.5 using potassium dihydrogen phosphate. Then, 5 g of nicotinamide mononucleotide was added, and pure water was added to a total of 500 ml with stirring until completely homogeneous.

### Test 1 - Efficiency comparison between the shampoo containing nicotinamide mononucleotide and the control shampoo

Sixteen male and sixteen female test subjects, aged 50 years or older, were recruited. Prior to testing, the hair color, growth rate, dandruff status, and hair distribution of the participants were assessed and scored by the researchers (Tables 1-4, and Methodology Table 12, and they were required to confirm that they had not undergone hair coloring, hair perming, or negative ion hair treatment within the past year. The participants also should commit to keeping away from the aforementioned things within 90 days after starting the study. And in case of hair trimming or hair care, the participants should contact the program director to make appropriate study arrangements, including reassessment.

The study was conducted in two batches by gender, and the shampoos prepared according to Example 1 or 13 above were distributed randomly by the researchers in a double-blind trial. The study required that the participants should wash their hair with the designated shampoo at least once every 24 hours, 2-5 ml of shampoo each time, with the shampoo and foam remaining on head for at least 2 minutes before being rinsed off with warm water. The test subjects were evaluated every 30 days, and the entire test lasted 90 days. The test results are shown in Tables 5-6 below.

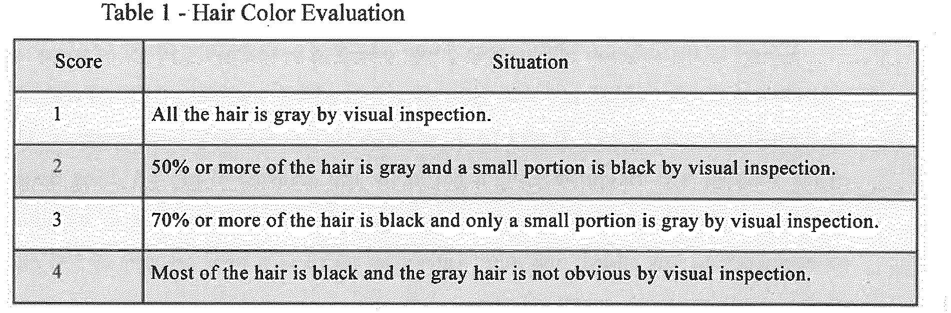

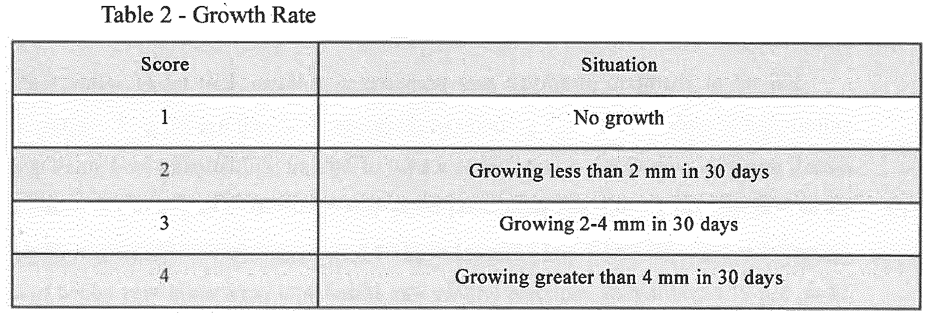

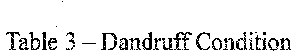

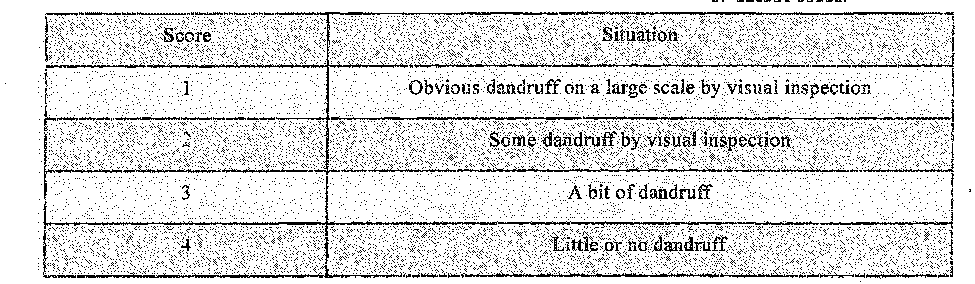

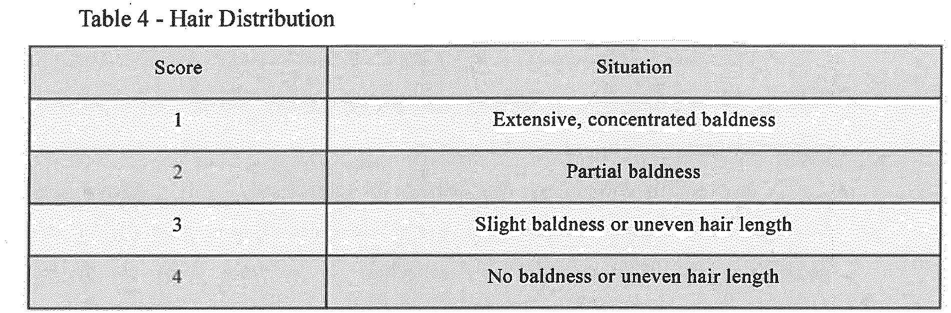

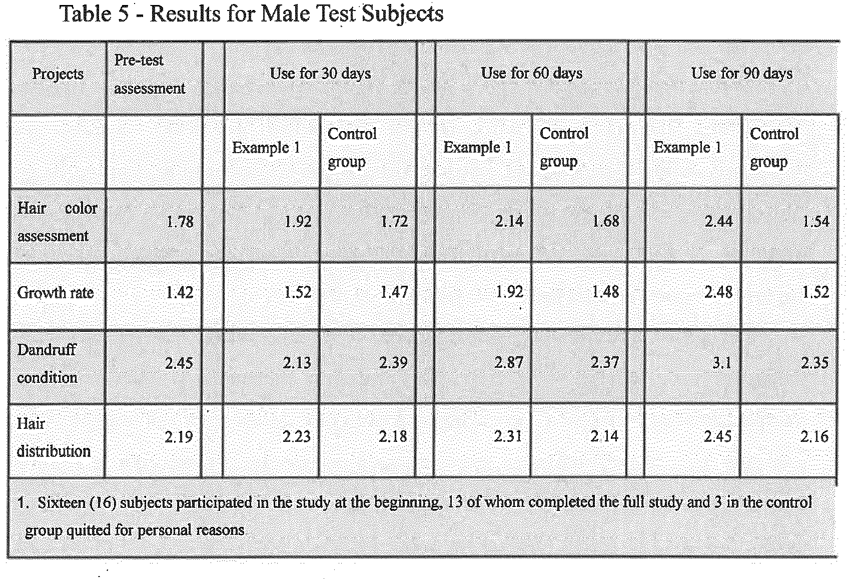

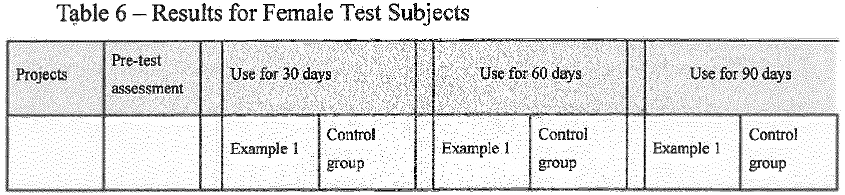

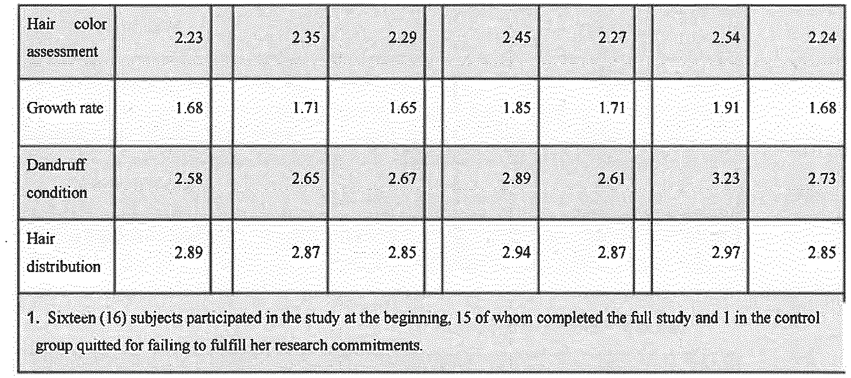

### Test 2 - Comparison of the effectiveness of the hair serum containing nicotinamide mononucleotide with the control hair serum

A hair serum containing nicotinamide mononucleotide was prepared according to Example 4 and a hair serum prototype was prepared according to Example 14, respectively. Eight men and eight women were recruited as the participants for the study. The participants should be in good health and aged 50 years or older. Before the test, the hair color, growth rate, dandruff condition and hair distribution of the participants were evaluated and scored by the researchers (referring to Tables 1-4), and they were required to confirm that they had not undergone hair coloring, hair perming, negative ion hair treatment or anything else that would influence the hair growth, including surgeries and therapy treatments, within the past year. Participants also should commit to keeping away from the above-mentioned things within 90 days after starting the study. And in case of hair trimming or hair care, the participants should contact the program director to make appropriate study arrangements, including re-evaluation.

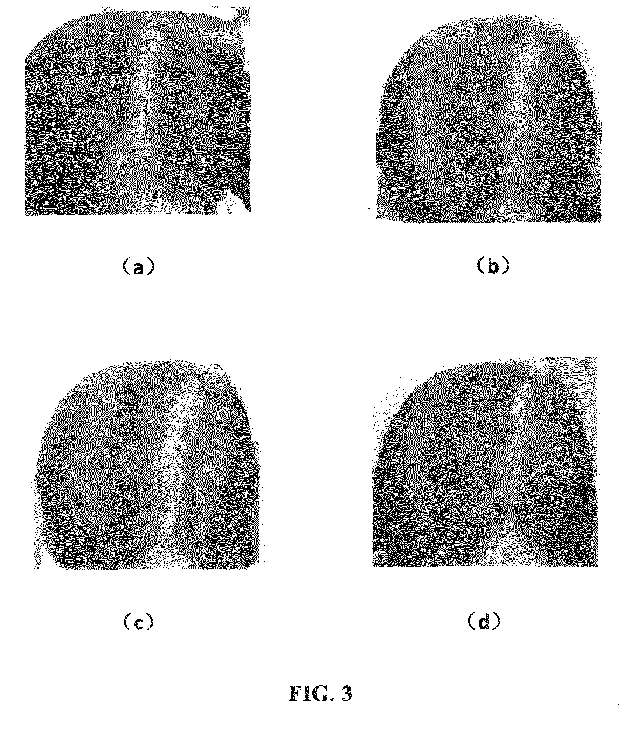

The study was conducted in two batches by gender, and the participants were provided randomly in a double-blind trial with either a hair serum containing nicotinamide mononucleotide prepared as in Example 4 above or a hair serum prototype prepared as in Example 14. The test subjects were required to use the hair serums twice a day in the morning and at night by the following procedure: immediately after washing hair and drying it with only a dry cloth but not a hot air dryer, applying 5-10 ml of the hair serum to the scalp with massaging for no less than five minutes to distribute the serum evenly and achieve complete absorption of the serum by the scalp. The test subjects were evaluated before the study and every 30 days after starting the study, and the entire study lasted 90 days. The test results are shown in Tables 7-9 and FIGs. 3-4 below.

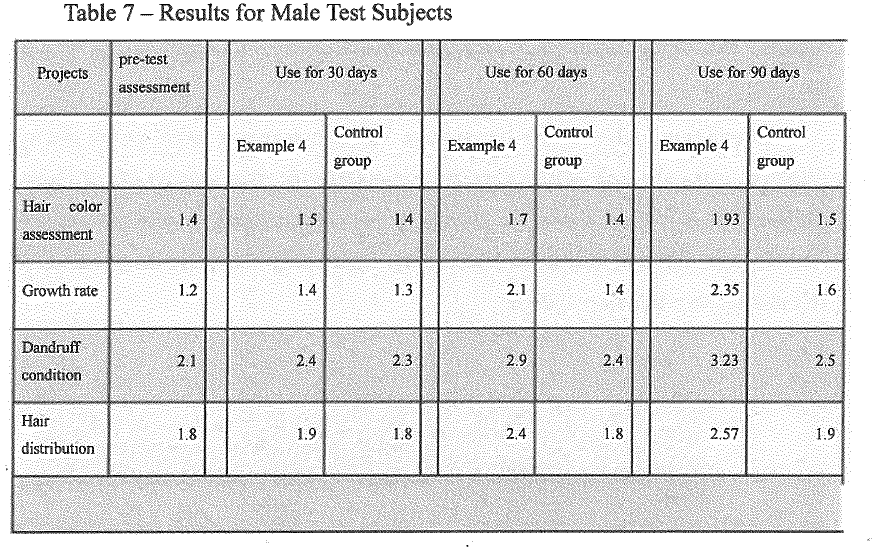

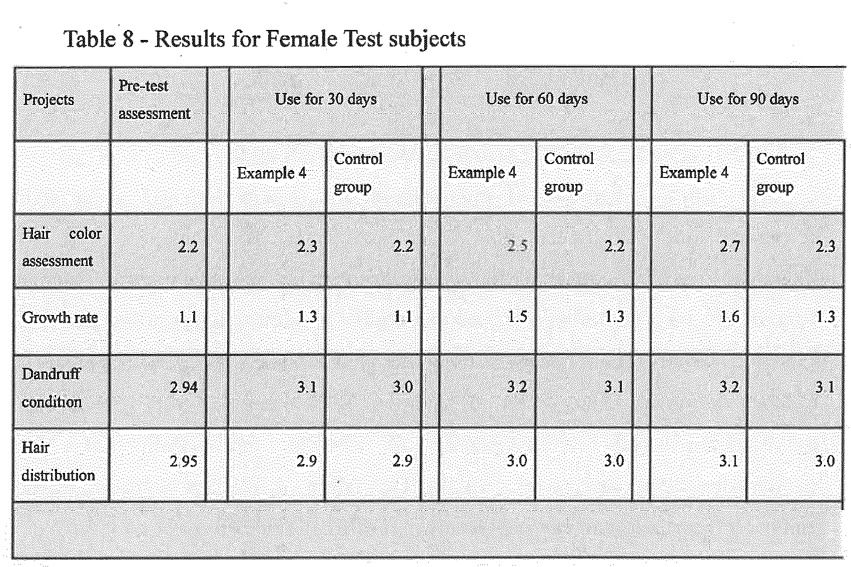

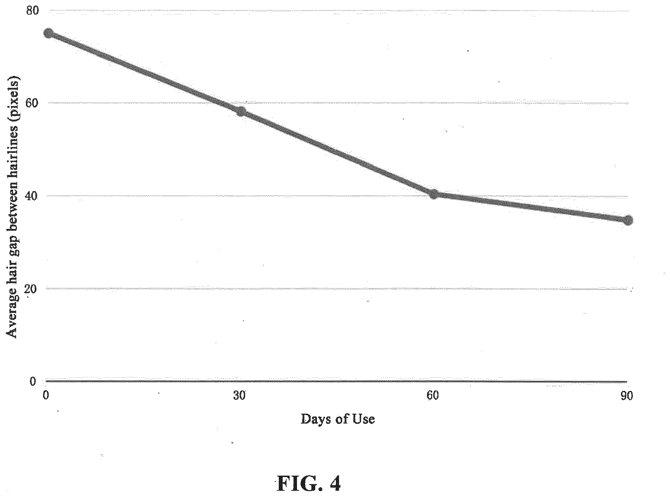

The hair growth of the female test subjects was also photographed and the hair growth density was calculated. The results are shown in FIGs. 3(a)-(d), FIG. 4 and Table 9. FIGs. 3(a)-(d) are photographs showing the hair growth condition of female test subjects using the hair serum containing nicotinamide mononucleotide of Example 4 on different days. Specifically, the photographs demonstrate the effect of the hair serum on the female test subjects within 90 days; wherein the red line is the hair axis, and the average gap between hairlines is the distance between the hairs on both sides measured by taking six points on the axis using Adobe Photoshop CS software. A larger distance may be regarded as a sparser hair distribution, and a smaller distance may be regarded as a thicker hair distribution.

In addition, Table 9 lists the average hair gap between hairlines for the female test subjects using the hair serum containing nicotinamide mononucleotide of Example 4 on different days. FIG. 4 shows the change in the average hair gap between hairlines for the female test subjects using the hair serum containing nicotinamide mononucleotide of Example 4 as a function of days.

**Table 9**

| **Days of use** | **Average hair gap (pixels) between hairlines** |
|---|---|
| 0 | 75.02 |
| **30** | 58.18 |
| **60** | 40.37 |
| **90** | 34.86 |

### Test 3: Test on stability of nicotinamide mononucleotide

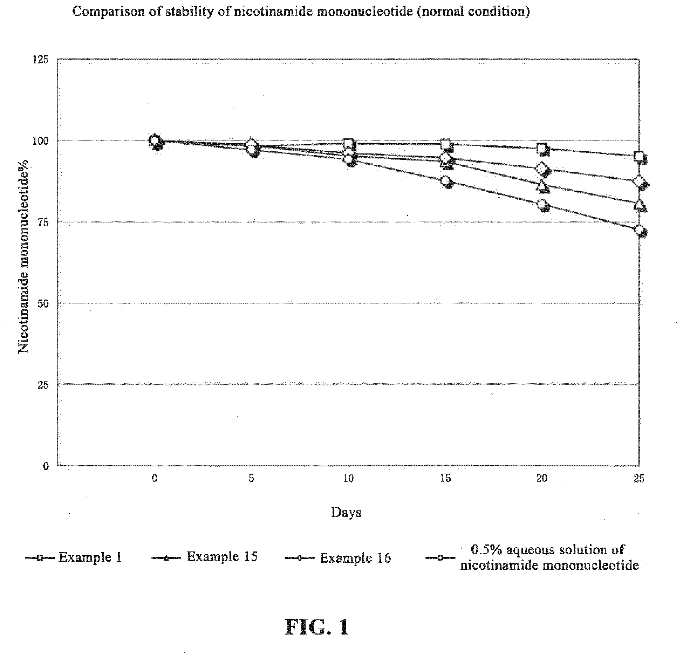

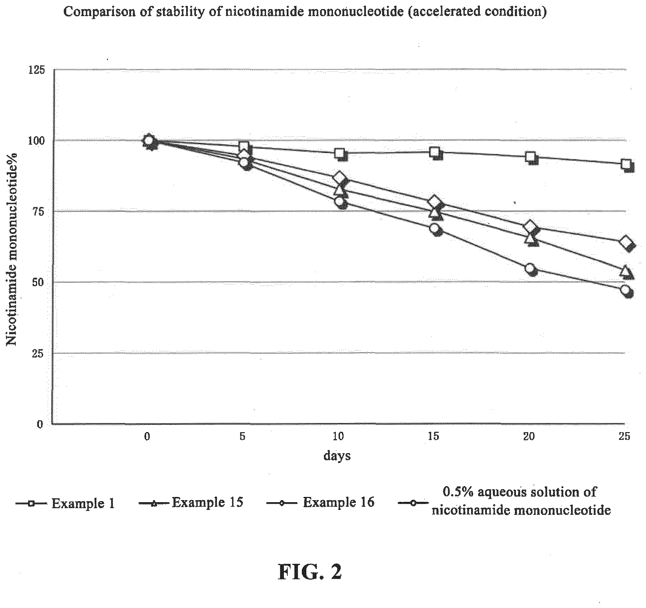

The aqueous solution of nicotinamide mononucleotide (2.5 g of nicotinamide mononucleotide dissolved in 500 ml of pure water) was compared with Example 1, Example 15 and Example 16 for testing the stability of nicotinamide mononucleotide. Specifically, the samples were placed in a normal condition (temperature: about 22 degrees Celsius; humidity: about 65%) and an accelerated condition (temperature: about 37 degrees Celsius; humidity: about 85%). A study of 30-day duration was carried out by high performance liquid chromatography to observe the stability of nicotinamide mononucleotide in each group over days. The stability results can be found in FIGs. 1 and 2, and the test methods and instruments are described in Table 10.

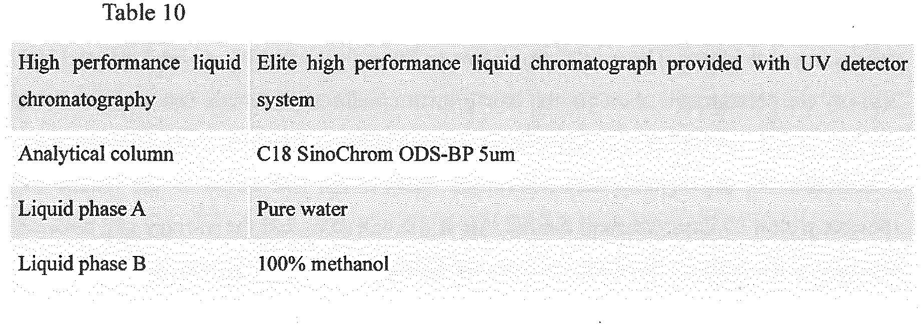

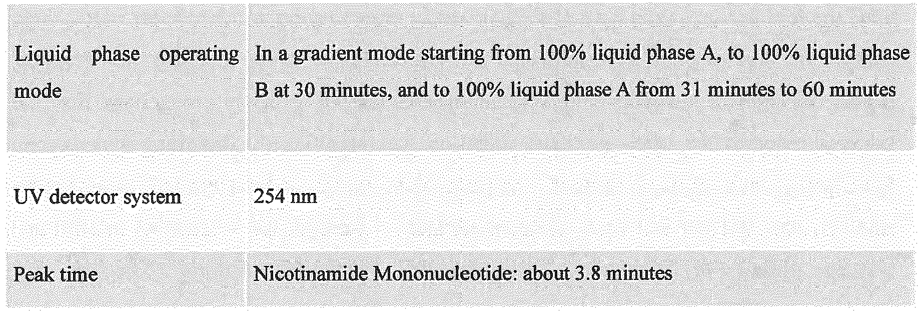

### Control 1: Administration of oral nicotinamide mononucleotide supplements only

The trial subjects were dosed with oral nicotinamide mononucleotide supplements (obtained from GeneHarbor (Hong Kong) Biotechnologies Ltd., brand name: NMN9000), by taking two capsules with a total of 300 mg every morning after breakfast. The trial subjects were subjected to questionnaires survey after three months to assess their physical health, hair growth rate, hair color change, and the number of newly grown hair. The trial subjects reported significant improvements in mental state, physical strength, sleep quality and skin. Specifically, the trial subjects reported that their deep sleep time was prolonged, their exercise capacity and recovery speed were improved, and they had not experienced any physical pain or symptoms in the past three months. Also, the dry mouth, irritation, intermittent mental discomfort, chills and fever often suffered by the older people were greatly reduced, and the personal sense of both hearing and vision was improved and became more sensitive than before. During the test, the researchers found a great change that a small amount of black hair was growing on the back of the head near the neck that was previously occupied by white hair three months before. Hair growth rate, hair color change, and contrast ratio of newly grown hair were slightly improved after observation compared with the case of three months before, but such improvement was relatively slow compared to the case where the nicotinamide mononucleotide-containing shampoo for neutral hair was used and the case where the hair serum containing nicotinamide mononucleotide was used. The trial subjects believed that the reduction of hair loss was the most obvious effect after taking the supplements, while the itchy scalp and dandruff were significantly reduced.

### Control 2: Administration of nicotinamide mononucleotide supplements combined with use of the hair serum containing nicotinamide mononucleotide

The trial subjects were dosed with oral nicotinamide mononucleotide supplements (obtained from GeneHarbor (Hong Kong) Biotechnologies Ltd., brand name: NMN9000), by taking two capsules with a total of 300 mg every morning after breakfast. At the same time, the trial subjects used 5 ml of the hair serum after washing hair every day. After three months, the trial subjects were subjected to a questionnaire survey to assess their physical health, hair growth rate, hair color change and the number of newly grown hair. The trial subjects reported that their physical condition was significantly improved than before. Specifically, their physical strength was better than before, and the most obvious change was that they did not feel short of breath or lack of strength when climbing the hill and walking upstairs or downstairs. Their skin was tighter than before. Moreover, they experienced shorter sleep time than before but woke up full of energy, and would not sleep in or go back to sleep again. The frequency of insomnia was reduced and the frequent urination was improved, such that the quality of life was improved a lot. The hair growth rate, hair color change, and contrast ratio of newly grown hair after observation were significantly different from the case three months before. Particularly, in the hair that was white previously, newly grown black hair could be seen obviously, accounting for about 20% of the hair. The researchers took ten white hairs and ten black hairs for careful study under the electron microscope, and made the following conclusions:

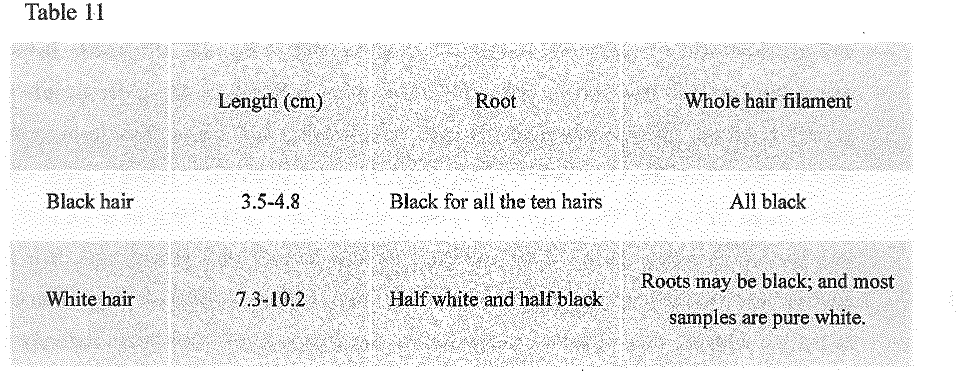

Compared to taking the oral nicotinamide mononucleotide supplement alone, this subject experienced more pronounced hair growth and more black hairs were found in the hairs that grew.

It should be understood that the application of the present invention is not limited to the above-mentioned embodiments. For those of ordinary skill in the art, improvements or modifications can be made according to the above description, and all such improvements and modifications should fall within the protection scope of the appended claims of the present invention.

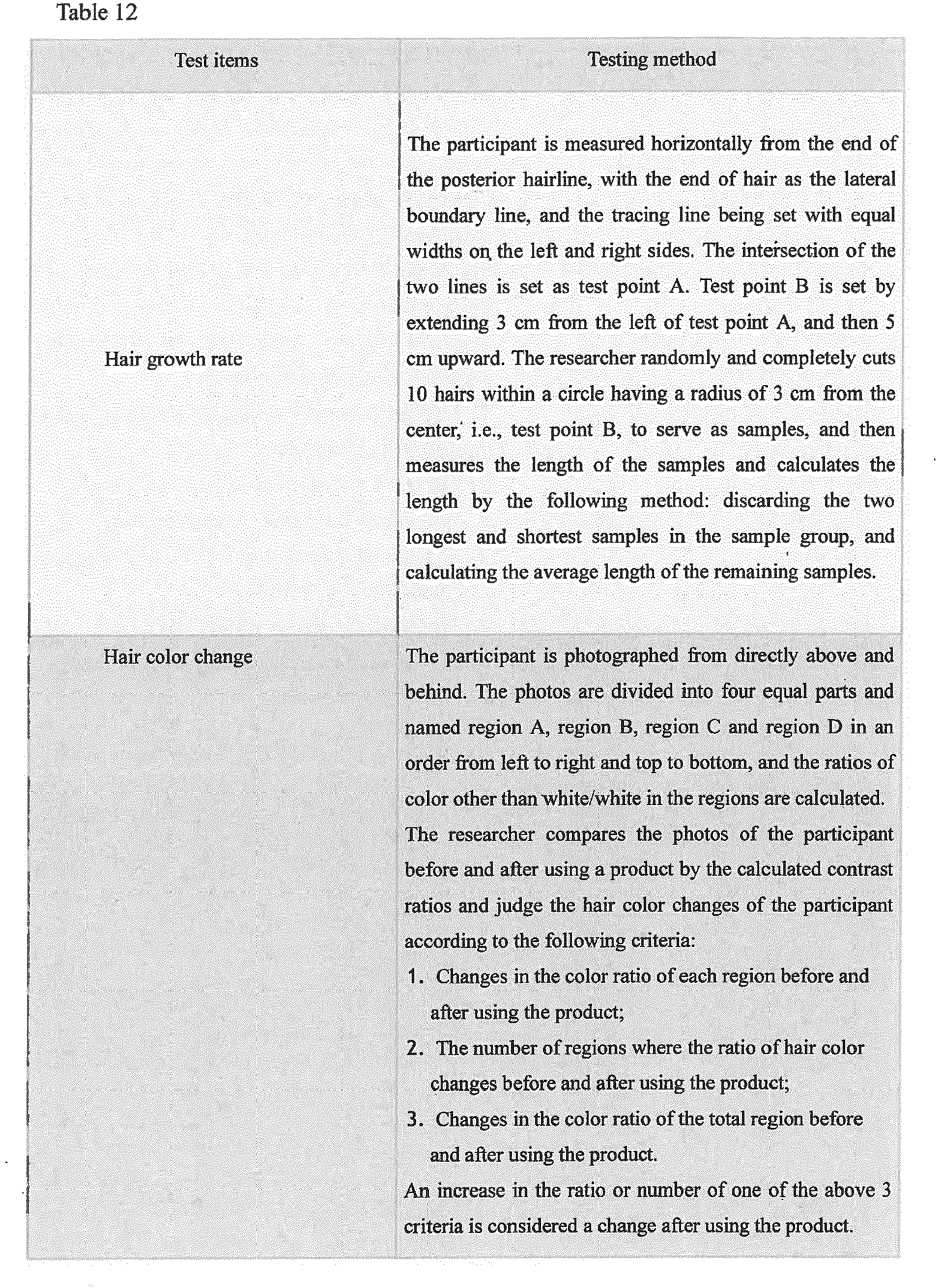

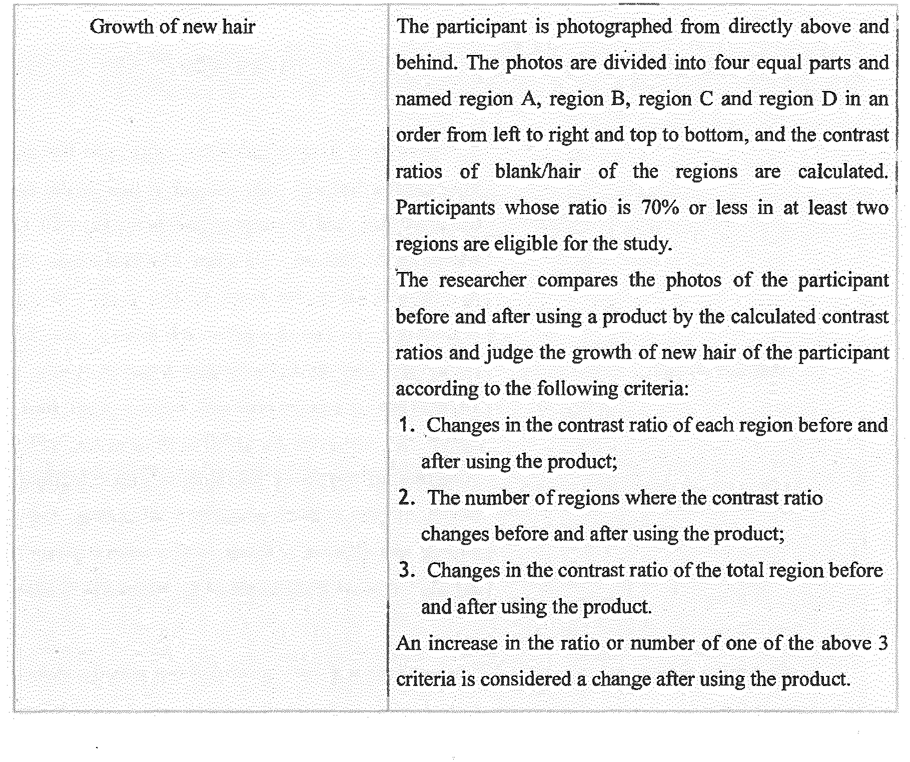

## Claims

1. A hair care composition **characterized by** comprising nicotinamide mononucleotide or a salt thereof or a hydrate thereof, and a base ingredient for stabilizing the nicotinamide mononucleotide or the salt thereof or the hydrate thereof.

2. The hair care composition according to claim 1, **characterized in that** the nicotinamide mononucleotide or the salt thereof or the hydrate thereof is in solid, liquid or atomized form.

3. The hair care composition according to claim 1 or 2, **characterized in that** the base ingredient for stabilizing the nicotinamide mononucleotide or the salt thereof or the hydrate thereof comprises one or more of an anionic surfactant, an amphoteric surfactant, a cationic surfactant, an antioxidant, a pH buffer and an acidity regulator.

4. The hair care composition according to any one of claims 1-3, **characterized in that** the hair care composition is in the form of any one of a shampoo, conditioner, serum, soap, spray, cream, hair wax, gel and paste.

5. The hair care composition according to any one of claims 1-4, **characterized in that** the hair care composition is any one of an eyebrow care product, an eyelash care product and a beard care product.

6. The hair care composition according to claim 1, **characterized in that** the base ingredient comprises an ingredient for preventing degradation of nicotinamide mononucleotide in liquid or atomized form, such as one or more selected from sialic acid, theanine, beta-carotene and sodium phosphate.

7. The hair care composition according to any one of claims 1-6, **characterized in that** the nicotinamide mononucleotide or the salt thereof or the hydrate thereof represents from 0.01 wt% to 25 wt%, preferably from 0.1 wt% to 20 wt%, and more preferably from 0.1 wt% to 15 wt% of the total weight of the hair care composition, based on nicotinamide mononucleotide.

8. The hair care composition according to any one of claims 1-7, **characterized in that** the hair care composition further comprises water, and
optionally, the water is present in an amount of 50-95 wt%, preferably 60-92 wt%, and more preferably 65-90 wt%, based on the total weight of the hair care composition.

9. The hair care composition according to claim 3, **characterized in that**, relative to the total weight of the hair care composition,
the anionic surfactant is present in an amount of 15-65 wt%, preferably 25-45 wt%, and more preferably 30-40 wt%;
optionally, the amphoteric surfactant is present in an amount of 1-15 wt%, preferably 1.5-8 wt%, and more preferably 2-7 wt%;
optionally, the cationic surfactant is present in an amount of 0.01-0.09 wt%, preferably 0.02-0.08 wt%, and more preferably 0.03-0.08 wt%;
optionally, the antioxidant is present in an amount of 0.5-3 wt%, preferably 0.9-2.5 wt%, and more preferably 1.5-2 wt%;
optionally, the acidity regulator is present in an amount of 0.05-1 wt%, preferably 0.2-0.85 wt%, and more preferably 0.3-0.7 wt%; and
optionally, the pH buffer is present in an amount of 1-3 wt%, preferably 1.5-2.5 wt%, and more preferably 2-2.5 wt%.

10. The hair care composition according to claim 3 or 9, **characterized in that** the anionic surfactant is selected from at least one of sodium laureth sulfate, sodium lauryl sulfate, ammonium lauryl sulfate, sodium methyl cocoyl taurate, lauryl polyether-5 carboxylic acid, sodium lauryl glucose carboxylate and mixtures thereof.

11. The hair care composition according to claim 3 or 9, **characterized in that** the amphoteric surfactant is at least one of an alkyl amphoteric carboxylate, an alkyl amino acid salt, cocamidopropyl betaine, cocamide monoethanolamine, lauryl glucoside, coco methyl dextran or mixtures thereof.

12. The hair care composition according to claim 3 or 9, **characterized in that** the cationic surfactant is at least one selected from polyquaternium-10, guar hydroxypropyltrimethylammonium chloride or mixtures thereof.

13. The hair care composition according to claim 3 or 9, **characterized in that** the antioxidant is at least one selected from L-cysteine, DL-methionine, vitamin A, vitamin C, vitamin E, sodium phytate or mixtures thereof.

14. The hair care composition according to claim 3 or 9, **characterized in that** the acidity regulator is at least one selected from citric acid, oxalic acid, lactic acid, hydrochloric acid or mixtures thereof.

15. The hair care composition according to claim 5, wherein the eyebrow care product is an eyebrow care liquid, an eyebrow care spray or an eyebrow care cream; the eyelash care product is an eyelash care liquid assembly; and the beard care product is a beard care liquid, a beard care spray or a beard care cream.

16. The hair care composition according to any one of claims 1-15, **characterized in that** the hair care composition further comprises a functional additive or additives for enriching appearance, texture, odor and user experience of the hair care composition.

17. The hair care composition according to claim 16, **characterized in that** the functional additive or additives is/are selected from a sensitizer, a foam enhancer, a fragrance agent, a functional excipient or any combinations thereof.

18. A method for preparing a hair care composition, **characterized in that** the method comprises the steps of
providing nicotinamide mononucleotide or a salt thereof or a hydrate thereof,
providing an aqueous solution of a base ingredient for stabilizing the nicotinamide mononucleotide or the salt thereof or the hydrate thereof in a liquid or gaseous state; and
mixing the nicotinamide mononucleotide or the salt thereof or the hydrate thereof with the aqueous solution and adjusting the pH to obtain the hair care composition.

19. The method according to claim 18, **characterized in that** the base ingredient for stabilizing the nicotinamide mononucleotide or the salt thereof or the hydrate thereof in the liquid or gaseous state comprises one or more of an anionic surfactant, an amphoteric surfactant, a cationic surfactant, a nonionic surfactant, an antioxidant, a pH buffer and an acidity regulator.

20. The method according to claim 19, **characterized in that** the method comprises:
(1) mixing one or more anionic surfactants to obtain a first type of surfactant,
(2) mixing one or more amphoteric surfactants to obtain a second type of surfactant,
(3) optionally mixing one or more cationic surfactants to obtain a third type of surfactant,
(4) mixing the first type of surfactant, the second type of surfactant and optionally the third type of surfactant to obtain a complex surfactant,
(5) adding nicotinamide mononucleotide to the complex surfactant to obtain a mixture,
(6) adding a pH buffer to the mixture and adjusting the mixture to achieve a pH of 4.85-7.0 using an acidity regulator so as to obtain a hair care composition; and
(7) adding an antioxidant to the mixture, thereby obtaining the hair care composition.
